# EUROPÄISCHE PATENTSCHRIFT

(11) **EP 3 666 233 B1**
(45) Veröffentlichungstag und Bekanntmachung des Hinweises auf die Patenterteilung: **24.04.2024**
(21) Anmeldenummer: 19212043.4
(22) Anmeldetag: 28.11.2019
(51) Int. Cl.: A61F 5/01, A61F 5/10, A61F 5/37, A61F 2/78

(54) **THERAPIEHANDSCHUH UND FIXIERSCHIENE FÜR EINEN THERAPIEHANDSCHUH**
THERAPEUTIC GLOVE AND FIXING RAIL FOR A THERAPY GLOVE
GANT THÉRAPEUTIQUE ET RAIL DE FIXATION POUR GANT THÉRAPEUTIQUE

(30) Priorität: 30.11.2018 DE 102018130567
(43) Veröffentlichungstag der Anmeldung: 17.06.2020
(73) Patentinhaber: Meyer-Clasen, Petra, 78052 Villingen-Schwenningen (DE)
(72) Erfinder: Meyer-Clasen, Carsten, 78126 Königsfeld (DE)
(74) Vertreter: Mammel und Maser Patentanwälte PartG mbB

(56) Entgegenhaltungen:
- BE-A- 904 432
- DE-A1-102013 004 714
- DE-B3-102005 014 470
- DE-U1- 8 804 564
- US-A1- 2008 103 424

## Beschreibung

Die Erfindung betrifft einen Therapiehandschuh zur Fixierung von zumindest einem Finger sowie eine Fixierschiene für einen solchen Therapiehandschuh.

Aus der DE 20 2007 002 380 U1 ist ein Therapiehandschuh zur Fixierung von zumindest einem Finger bekannt. Solche Therapiehandschuhe werden insbesondere zur nächtlichen Strecklagerung von zumindest einem Finger bei einer Dupuytren-Krankheit oder bei einem Dupuytren-Knotenbefall eingesetzt. Ein solcher Therapiehandschuh umfasst einen Zentralbereich, der den Handrücken und die Handfläche umschließt. Ausgehend von dem Zentralbereich sind Fingerlinge vorgesehen, in welchen der jeweils zu fixierende Finger aufgenommen werden kann. Der Therapiehandschuh weist eine Fixierschiene auf, welche einen Haltezentralbereich und davon ausgehend einzelne Fingerschienenabschnitte aufweist. Zur Fixierung der Fixierschiene an dem Therapiehandschuh sind an den Fingerlingen Fixiertaschen vorgesehen, in welchen die Fingerschienenabschnitte einsetzbar sind. Des Weiteren ist ein lösbarer Klettverschluss im Zentralbereich vorgesehen, um den Hauptschienenabschnitt zum Zentralbereich des Handschuhs lösbar zu fixieren. Durch diese Anordnung ist ermöglicht, dass der im Fingerling aufgenommene Finger in einer Therapiestellung relativ zum Handrücken fixiert ist. Da die Fingerlinge aus einem atmungsaktiven und flexiblen Material bestehen, kann die Strecklagerung des Fingers nicht vollständig aufrechterhalten werden.

Aus der DE 20 2011 104 828 U1 ist des Weiteren ein Therapiehandschuh zur Fixierung von zumindest zwei Fingern bekannt. Dieser Therapiehandschuh umfasst einen Handschuh mit zwei daran anordenbaren Fixierschienen. Eine erste Fixierschiene dient zur Positionierung des kleinen Fingers, des Ringfingers und des Mittelfingers in einer Strecklage. Die zweite Fixierschiene erstreckt sich zwischen dem Zeigefinger und dem Daumen. Die Fixierschienen sind ebenfalls über Klettverschlüsse lösbar und abnehmbar mit dem Handschuh verbunden. Bei dieser Ausführungsform ist die Fixierschiene einer Handfläche zugeordnet. Dieser Fixierhandschuh weist den Nachteil auf, dass die Strecklagerung weder eingestellt noch aufrechterhalten werden kann.

Aus der BE 904 432 A ist ein Therapiehandschuh zur Fixierung von einem Finger bekannt. Dieser Therapiehandschuh umfasst einen Zentralbereich, welcher einen Handrücken und eine Handfläche umschließt. An diesem Zentralbereich ist eine Fixierschiene befestigbar. Am freien Ende der Fixierschiene ist ein Klettband zur Fixierung einer Fingerspitze vorgesehen. BE 904 432 A zeigt einen Therapiehandschuh gemäß dem Oberbegriff des Anspruchs 1.

Aus der DE 88 04 564 U1 ist eine Prothese zur konservativen Behandlung der Dupuytrenschen Kontraktur bekannt. Diese Prothese besteht aus einer Handgelenksstütze mit einer Daumenschlaufe. An den Fingern werden steife Lederhülsen befestigt, die mit Hilfe von straffen Leinenbändern an einem Klettverschluss der Handgelenksstütze befestigt werden.

Der Erfindung liegt die Aufgabe zugrunde, einen Therapiehandschuh sowie eine Trägerschiene für einen Therapiehandschuh vorzuschlagen, bei welchem eine verbesserte Therapie ermöglicht ist. Diese Aufgabe wird durch einen Therapiehandschuh gelöst, bei welchem an jedem Fingerling, der eine Fixiertasche zur Aufnahme einer Fixierschiene aufweist, ein lösbarer Verschluss vorgesehen ist, durch welchen eine Anlageposition des Fingers im Fingerling zum Fingerschienenabschnitt einstellbar ist. Durch diesen lösbaren Verschluss wird ermöglicht, dass der Finger unmittelbar an die Schiene gedrückt wird oder daran anliegend positioniert wird. Diese anliegende Position des Fingers an der Trägerschiene kann durch den Verschluss aufrechterhalten bleiben.

Bevorzugt ist vorgesehen, dass der Fingerling zumindest abschnittsweise aus einem elastisch nachgiebigen Material ausgebildet ist und die an dem Fingerling angeordnete Fixiertasche aus einem, vorzugsweise gegenüber dem Material des Fingerlings, unelastischen Material besteht. Diese Ausgestaltung weist den Vorteil auf, dass eine definierte Aufnahme der Fingerschiene bzw. Fingerschienenabschnitte in der jeweiligen Fixiertasche ermöglicht ist. Zum anderen ist durch die zumindest abschnittsweise aus einem elastisch nachgiebigen Material ausgebildeten Fingerlinge ein einfaches Anziehen des Therapiehandschuhs ermöglicht. Vorteilhafterweise ist der Querschnitt des Fingerlings mit dem zumindest abschnittsweise ausgebildeten elastischen nachgiebigen Material um eine halbe oder eine ganze Konfektionsgröße größer ausgebildet, als dies im Bereich der Handfläche vorgesehen ist.

Des Weiteren ist bevorzugt vorgesehen, dass der Fingerling einen der Fixiertasche gegenüberliegenden Bereich aus einem unelastischen Material aufweist, welcher zur Handinnenseite oder zur Handaußenseite ausgerichtet ist und zwischen dem unelastischen Bereich des Fingerlings und der Fixiertasche jeweils ein elastisch nachgiebigerer Bereich ausgebildet ist. Dadurch können die zur Handaußenseite und Handseiteninnenseite weisenden Bereiche, welche durch die Fingertasche und den unelastischen Bereich des Fingerlings gebildet sind, robust und widerstandsfähig ausgebildet sein, wohingegen die dazwischenliegenden Bereiche elastisch nachgiebig sind, so dass wiederum ein einfaches Anlegen des Therapiehandschuhs ermöglicht bleibt. Durch den lösbaren Verschluss am Fingerling kann nach dem erleichterten Anlegen des Therapiehandschuhs die Anlageposition des Fingers im Fingerling zum Fingerschienenabschnitt definiert werden. Der zunächst vergrößerte Freiraum im Fingerling durch den elastisch nachgiebigen Bereich für das erleichterte Anlegen des Therapiehandschuhs kann im Umfang durch den lösbaren Verschluss reduziert werden. Dadurch liegt der Finger wiederum in Abhängigkeit der Therapie fest an dem Fingerschienenabschnitt an.

Des Weiteren ist bevorzugt vorgesehen, dass der lösbare Verschluss im Bereich des ersten oder zweiten Fingergliedes oder im Bereich des Gelenkes zwischen dem ersten und zweiten Fingerglied oder das erste Fingerglied und das sich daran anschließende Gelenk übergreifend vorgesehen ist. Dadurch kann in einfacher Weise ein verbesserter Kontakt zwischen dem Finger und der Schiene erfolgen. Zudem kann der Fingerling in seinem Material unverstärkt wie bislang ausgebildet sein.

Bevorzugt ist vorgesehen, dass der lösbare Verschluss ein Klettverschluss ist. Dies ermöglicht eine einfache Handhabung. Zudem ist ein solcher Klettverschluss robust, so dass ein solcher Therapiehandschuh auch für die Reinigung in einer Waschmaschine geeignet ist.

Des Weiteren ist bevorzugt vorgesehen, dass jeweils ein Ende des Haken- und Schlaufenbands fest an dem unelastischen Bereich des Fingerlings oder der Fixiertasche vorgesehen ist und die gegenüberliegenden Enden an der Fixiertasche oder dem unelastischen Bereich des Fingerlings zueinander fixiert werden und den Klettverschluss bilden. Diese Ausführungsform ermöglicht, dass jeweils ein Band seitlich entlang des elastisch nachgiebigen Bereichs des Fingerlings geführt wird, so dass ein Abstand zwischen dem unelastischen Bereich an dem Fingerling und der Fixiertasche verringert und somit der Finger zum Fingerschienenabschnitt fixiert gehalten wird.

Gemäß einer alternativen Ausführungsform ist vorgesehen, dass der Klettverschluss eine Lasche aufweist, an deren einem Ende das Haken- oder Schlaufenband vorgesehen ist, wobei das eine Ende der Lasche an der Fixiertasche oder dem nicht elastischen Bereich des Fingerlings fixiert, insbesondere angenäht oder angeklebt ist, und das gegenüberliegende Ende der Lasche, welches das komplementäre Schlaufen- oder Hakenband umfasst, mit dem ersten Ende der Lasche verbindbar ist. Dadurch kann die Lasche einmal um den Fingerling mit der Fixiertasche herumgeführt werden, um die Lasche zu fixieren. Dies ermöglicht eine einer Ein-Hand-Betätigung.

Das Haken- und Schlaufenband kann gemäß einer ersten Ausführungsform an einer Fingeraußenseite vorgesehen sein. Alternativ kann das Haken- und Schlaufenband auch an einer Fingerinnenseite fixierbar sein, so dass der lösbare Verschluss gegenüberliegend der Fixierung des Haken- und Schlaufenbereichs liegt.

Des Weiteren kann vorgesehen sein, dass der lösbare Verschluss gegenüberliegend zur Fixiertasche für die Fixierschiene an dem Fingerling vorgesehen ist. Auch eignet sich eine Anordnung des lösbaren Verschlusses unmittelbar an der Fixiertasche, in welche der Fingerschienenabschnitt einsteckbar ist.

Vorteilhafterweise weist der Therapiehandschuh eine Fixierschiene mit zumindest einem Fingerschienenabschnitt auf, welcher eine längliche Aussparung aufweist. Durch diese längliche Aussparung kann der Kontakt des Fingers zur Schiene weiter verbessert werden. Eine Innenseite des Fingers kann geringfügig in die Aussparung eingreifen, so dass der Finger durch beidseitige Schienen, die durch die Aussparung im Fingerschienenabschnitt gebildet sind, aufgenommen und geführt werden kann.

Die längliche Aussparung in dem Fingerschienenabschnitt folgt bevorzugt einer äußeren Kontur des Fingerschienenabschnitts. Dadurch wird ein umlaufender Steg gebildet. Dieser umlaufende Steg ist bevorzugt mit einer konstanten Breite ausgebildet. Dies stellt ein einfach herzustellendes Design dar. Zudem kann eine angenehme Auflage des Fingers für den Benutzer gegeben sein.

Des Weiteren ist bevorzugt vorgesehen, dass die Breite des Steges schmaler als die Breite der länglichen Aussparung ist. Dies weist neben dem Tragekomfort auch den Vorteil auf, dass ggf. eine Biegung an einem der Fingerschienenabschnitte in einfacher Weise eingebracht werden kann.

Eine alternative Ausgestaltung der länglichen Aussparung in dem Fingerschienenabschnitt sieht vor, dass in Längserstreckung gesehen zwei bauchige Konturen vorgesehen sind, welche im Bereich der Gelenke zwischen dem ersten und zweiten Fingerglied und dem zweiten und dritten Fingerglied vorgesehen sind. Dadurch kann im Bereich der Gelenke eine geringfügige Ausbauchung oder Verbreiterung der länglichen Aussparung gegeben sein, so dass der Tragekomfort weiter erhöht werden kann.

Des Weiteren ist bevorzugt vorgesehen, dass im Hauptschienenabschnitt der Fixierschiene zumindest eine Freinehmung vorgesehen ist. Dadurch kann eine weitere Gewichtseinsparung des Therapiehandschuhs ermöglicht und somit eine Verbesserung des Tragekomforts gegeben sein.

Die Fixierschiene bestehend aus dem Hauptschienenabschnitt und zumindest einem Fingerschienenabschnitt ist bevorzugt als Stanzteil ausgebildet. Dies ermöglicht eine einfache Herstellung. Insbesondere sind die Fixierschienen aus einem Aluminiumblechgestell. Bevorzugt sind die Fixierschienen mit einer Lackierung oder Beschichtung versehen. Alternativ kann die Fixierschiene auch aus Kunststoff, insbesondere einem durch Wärme verformbaren Kunststoff, hergestellt sein. Solche Trägerschienen können auch in einem 3-D-Druckverfahren aus einem metallischen Material oder aus Kunststoff hergestellt sein. Dadurch können in einfacher Weise individuelle Anpassungen an die Größe der Hand und der Finger des Benutzers ermöglicht sein. Des Weiteren kann vorgesehen sein, dass die Fixierschiene aus einem faserverstärkten Kunststoff, wie beispielsweise einem glasfaserverstärkten oder karbonfaserverstärkten Kunststoff, hergestellt ist.

Die der Erfindung zugrundeliegende Aufgabe wird des Weiteren durch eine Fixierschiene für einen Therapiehandschuh, nach einem der vorbeschriebenen Ausführungsformen, gelöst, bei welchen der zumindest eine Fingerschienenabschnitt eine längliche Aussparung aufweist. Dadurch kann eine verbesserte Anlage des Fingers an dem Fingerschienenabschnitt ermöglicht sein. Eine einzige linienförmige Auflage, die zu Druckstellen führen kann, wird verhindert. Die längliche Aussparung im Fingerschienenabschnitt der Fixierschiene verläuft bevorzugt entlang einer äußeren Kontur des Fingerschienenabschnitts. Dadurch kann ein umlaufender Steg gebildet werden, so dass der Finger an zwei zueinander beabstandeten Stegen aufliegen kann.

Die Breite des Steges am Fingerschienenabschnitt ist bevorzugt schmaler als die Breite der länglichen Aussparung gegeben. Dadurch kann der Tragekomfort verbessert sein.

insbesondere weist die Aussparung in den Fingerschienenabschnitten in Längserstreckung ein oder zwei bauchige Konturen auf, welche im Bereich der Gelenke zwischen dem ersten und zweiten Fingerglied und/oder dem zweiten und dritten Fingerglied vorgesehen sind.

Auch kann im Schienenabschnitt der Fixierschiene zumindest eine Freinehmung vorgesehen sein. Dies bildet eine Gewichtsreduzierung.

Gemäß einer Ausführungsform kann die Fixierschiene mit dem Hauptschienenabschnitt an dem zumindest einen Fingerschienenabschnitt als Stanzteil ausgebildet sein. Alternativ kann auch die Herstellung aus Kunststoff, insbesondere einem faserverstärkten oder durch Wärme verformbaren Kunststoff, vorgesehen sein.

Die Erfindung sowie weitere vorteilhafte Ausführungsformen und Weiterbildungen derselben werden im Folgenden anhand der in den Zeichnungen dargestellten Beispiele näher beschrieben und erläutert. Die der Beschreibung und den Zeichnungen zu entnehmenden Merkmale können einzeln für sich oder zu mehreren in beliebiger Kombination erfindungsgemäß angewandt werden. Es zeigen:
Figur 1 eine schematische Ansicht auf einen Therapiehandschuh mit einer Fixierschiene nach einem Anlegen an einer Patientenhand,
Figur 2 eine schematische Ansicht auf eine Innenseite des Therapiehandschuhs nach Figur 1,
Figur 3 eine schematische Ansicht auf die Fixierschiene im Therapiehandschuh gemäß Figur 1,
Figur 4 eine schematische Ansicht auf eine alternative Ausführungsform der Fixierschiene zu Figur 3,
Figur 5 eine schematische Ansicht einer alternativen Ausführungsform der Fixierschiene zu Figur 4,
[0033] Figur 6 eine schematische Schnittansicht entlang der Linie V-V in Figur 1,
Figur 7 eine schematische Schnittansicht einer alternativen Ausführungsform zu Figur 6, und
Figur 8 eine schematische Ansicht auf eine Innenseite eines Therapiehandschuhs gemäß einer alternativen Ausführungsform zu Figur 1 nach dem Anlegen an einer Patientenhand.

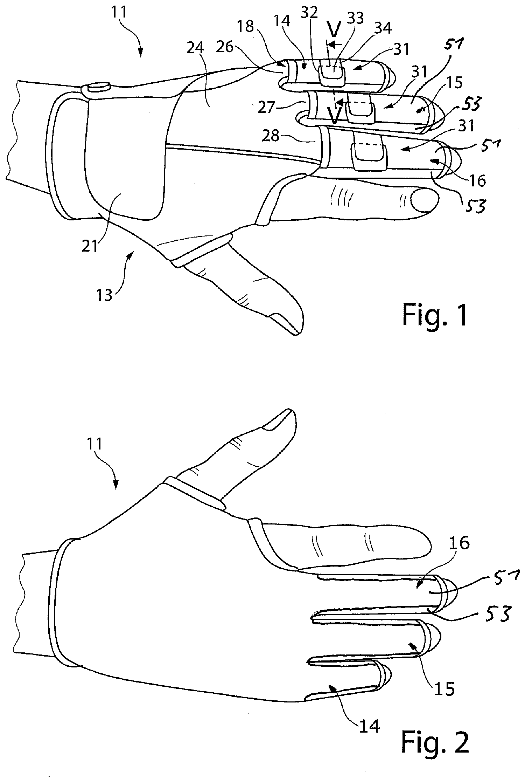

In Figur 1 ist eine erste Ausführungsform eines Therapiehandschuhs 11 dargestellt. Dieser Therapiehandschuh 11 ist an einer Hand eines Patienten angelegt. Der Therapiehandschuh 11 umfasst einen Handschuh 13 mit einem Zentralbereich 12, der sich sowohl am Handrücken gemäß Figur 1 als auch an der Handfläche gemäß Figur 2 erstreckt. Ausgehend von diesem Zentralbereich 12 erstrecken sich drei Fingerlinge 14, 15, 16. Diese dienen der Aufnahme des kleinen Fingers, des Ringfingers und des Mittelfingers.

Der Therapiehandschuh 11 weist an seiner Außenseite der Fingerlinge 14, 15, 16 Fixiertaschen 18 auf. Diese umfassen eine Einstecköffnung 19, welche zum Zentralbereich 12 ausgerichtet sind. Im Bereich des Handrückens ist ein lösbares Befestigungsmittel 21 vorgesehen.

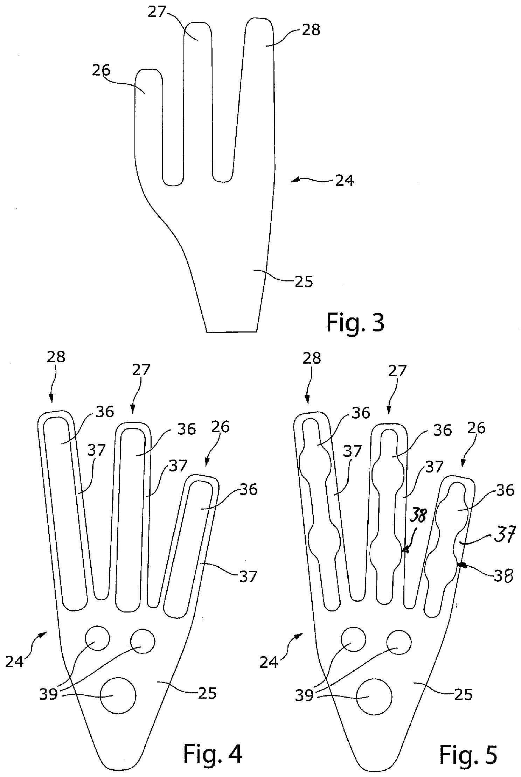

Eine Fixierschiene 24 gemäß Figur 3 umfasst einen Hauptschienenabschnitt 25, an welchen sich drei Fingerschienenabschnitte 26, 27, 28 erstrecken. Diese Fingerschienenabschnitte 26, 27, 28 weisen einen Verlauf auf, um dem kleinen Finger, Mittelfinger und dem Ringfinger zugeordnet zu werden. Eine solche Fixierschiene 24 ist aus einem plattenförmigen Material hergestellt. Bevorzugt kann es sich hierbei um ein Metallblech, insbesondere Aluminiumblech, handeln. Alternativ kann auch ein Kunststoff eingesetzt werden. Ebenso kann ein durch Wärme verformbarer Kunststoff oder ein faserverstärkter Kunststoff eingesetzt werden.

Diese Fixierschiene 24 ist im Therapiehandschuh 11 gemäß Figur 1 am Handrücken fixiert. Die Fingerabschnitte 26, 27, 28 werden in die Fixiertaschen 18 der Fingerlinge 14, 15, 16 eingesteckt. Darauffolgend kann der Hauptschienenabschnitt 25 durch das als lösbarer Verschluss ausgebildete Befestigungsmittel 21 fixiert gehalten werden.

Durch diese Anordnung wird ermöglicht, dass eine gewünschte therapeutische Streckstellung des oder der Finger mit der Fixierschiene 24 ermöglicht ist.

Zur Anlage des oder der Finger in dem oder den Fingerlingen 14, 15, 16 an dem jeweiligen Fingerschienenabschnitt 26, 27, 28 ist an jedem Fingerling 14, 15, 16 ein lösbarer Verschluss 31 vorgesehen. Dieser lösbare Verschluss 31 ist am Fingerling 14, 15, 16 im Bereich des ersten Fingergliedes angeordnet. Alternativ kann der lösbare Verschluss 31 auch im Bereich des Gelenkes zwischen dem ersten und zweiten Fingerglied vorgesehen sein. Auch kann der lösbare Verschluss 31 breit ausgebildet sein, so dass dies einen Teil des ersten Fingergliedes und das Gelenk zwischen dem ersten und zweiten Fingerglied überdeckt.

Der lösbare Verschluss 31 an den Fingerlingen 14, 15, 16 ist bevorzugt als Klettverschluss ausgebildet, welcher ein Hakenband 32 und ein Schlaufenband 33 umfasst. Das Hakenband 32 ist bevorzugt unmittelbar an dem Fingerling 14, 15, 16 fixiert, insbesondere angenäht oder angeklebt. Das Schlaufenband 33 ist an einer Lasche 34 vorgesehen, wobei die Lasche 34 entfernt vom Hakenband 32 am Fingerling 14, 15, 16 befestigt ist oder an einem Ende des Hakenbandes 32.

Es versteht sich, dass die Anordnung des Hakenbandes und Schlaufenbandes 32, 33 sowohl für das vorstehend beschriebene Beispiel als auch für die nachfolgenden Beispiele vertauscht angeordnet sein kann.

Durch die Anordnung des Schlaufenbandes 33 und der Lasche 34 wird ermöglicht, dass das Schlaufenband 33 in verschiedene Positionen zum Hakenband 32 angeordnet werden kann. Dadurch kann der Benutzer die Anlage des Fingers an dem Fingerschienenabschnitt 26, 27, 28 einstellen. Insbesondere ist dadurch sichergestellt, dass der Finger an dem Fingerschienenabschnitt 26, 27, 28 anliegt und nicht abheben kann.

Durch die Befestigung des Hakenbandes 32 an dem Fingerling 14, 15, 16 kann eine einfache Ein-Hand-Bedienung ermöglicht sein.

Bei der Ausführungsform gemäß Figur 1 ist die Fixierschiene 24 dem Handrücken zugeordnet. Ebenso ist der lösbare Verschluss 31 des jeweiligen Fingerlings 14, 15, 16 an einer Außenseite der Finger angeordnet. Alternativ kann vorgesehen sein, dass der lösbare Verschluss 31 auch an einer Innenseite des Fingerlings 14, 15, 16 vorgesehen ist.

Eine weitere Ausführungsform des Therapiehandschuhs 11, welche nicht dargestellt ist, kann darin bestehen, dass die Fixiertaschen 18 sowie das lösbare Befestigungsmittel 21 auf der Handinnenseite angeordnet sind. Die lösbaren Verschlüsse 31 an den Fingerlingen 14, 15, 16 können ebenfalls an der Innenseite vorgesehen sein oder aber wiederum zur einfachen Bedienung an der Außenseite der Fingerlinge 14, 15, 16.

In Figur 4 ist eine alternative Ausführungsform einer Fixierschiene 24 zu Figur 3 dargestellt. Diese Fixierschiene 24 umfasst einen Hauptschienenabschnitt 25, von welchem ausgehend sich Fingerschienenabschnitte 26, 27, 28 erstrecken. Beispielsweise sind drei Fingerschienenabschnitte 26, 27, 28 dargestellt. Auch können an dem Hauptschienenabschnitt 25 nur ein oder zwei Fingerabschnitte oder auch vier Fingerabschnitte vorgesehen sein.

In dem jeweiligen Fingerschienenabschnitt 26, 27, 28 ist eine längliche Aussparung 36 vorgesehen. Diese längliche Aussparung 36 erstreckt sich bevorzugt entlang dem gesamten Fixierschienenabschnitt 26, 27, 28. Dadurch weist der Fixierschienenabschnitt 26, 27, 28 im Vergleich zu der Ausführungsform in Figur 3 keine flächige Auflage für den jeweiligen Finger auf. Vielmehr ist ein umlaufender Steg 37 ausgebildet, welcher den Fingerschienenabschnitt 26, 27, 28 bildet. Bevorzugt ist dieser Steg 37 in einem Verlauf mit einer konstanten Breite vorgesehen.

In dem dargestellten Ausführungsbeispiel gemäß Figur 4 ist die Aussparung 36 länglich rechteckförmig mit runden Ecken ausgebildet. Alternativ kann die Aussparung 36 auch eine innere Kontur aufweisen. Beispielsweise kann diese bauchig sein, so dass ein innerer Rand der Aussparung 36 einer äußeren Kontur der Finger folgt. Beispielsweise können zwei bauchige Abschnitte vorgesehen sein im Bereich der Gelenke.

Vorteilhafterweise sind in dem Hauptschienenabschnitt 25 ein oder mehrere Freinehmungen 39 vorgesehen. Diese können in der Kontur, in der Anordnung und/oder in der Anzahl beliebig sein. Die Freinehmungen 39 dienen der Gewichtseinsparung.

In Figur 5 ist eine schematische Ansicht einer alternativen Ausführungsform der Fixierschiene 24 zu Figur 4 dargestellt. Bei dieser Ausführungsform ist vorgesehen, dass die Aussparung 36 zumindest eine, vorzugsweise zwei, bauchige Konturen 38 umfasst, welche im Bereich der Gelenke zwischen dem ersten und zweiten Fingerglied bzw. zweiten und dritten Fingerglied vorgesehen sind. Dadurch wird der Tragekomfort erhöht. Zudem kann ein verbessertes Anliegen des Fingers an der Fixierschiene 24 über den lösbaren Verschluss 31 eingestellt werden.

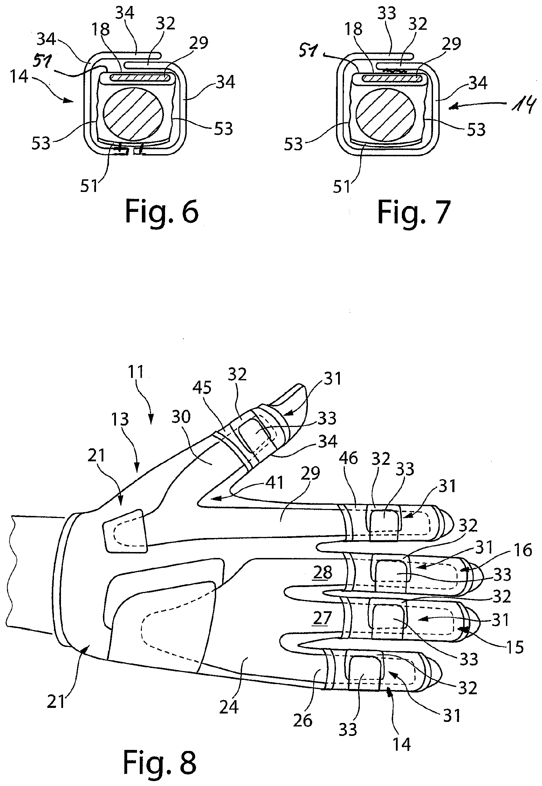

In Figur 6 ist eine schematische Schnittansicht entlang der Linie V-V in Figur 1 dargestellt. Bei dieser Ausführungsform ist vorgesehen, dass die Fixiertasche 18 aus einem unelastischen Material oder nicht nachgiebigen Material ausgebildet ist. Die Form der Fixiertasche 18 wird nach dem Einschieben des Fingerschienenabschnitts 26, 27, 28, 29, 30 beibehalten. An diese Fixiertasche 18 ist der Fingerling 14, 15, 16, 46, 47 angenäht. Dieser Fingerling 14, 15, 16, 46, 47 besteht zumindest abschnittsweise aus einem elastisch nachgiebigen Material. Beispielsweise kann der gesamte Fingerling 14, 15, 16, 46, 47 aus einem elastisch nachgiebigen Material bestehen, wodurch das Anlegen des Therapiehandschuhs vereinfacht ist. Bei der Ausführungsform gemäß Figur 6 ist bevorzugt vorgesehen, dass ein der Fixiertasche 18 gegenüberliegender Bereich 51 des Fingerlings 14, 15, 16 aus einem unelastischen Material oder nicht nachgiebigen Material besteht und die zwischen der Fixiertasche und dem unelastischen Bereich 51 sich erstreckende Abschnitte oder Bereiche 53 aus einem elastisch nachgiebigen Material bestehen. Auch bei dieser Ausführungsform kann ein erleichtertes Anziehen des Therapiehandschuhs 11 gegeben sein, da ein Aufweiten des Fingerlings zwischen der Fixiertasche 18 und dem gegenüberliegenden unelastischen Bereich 51 möglich ist.

Der unelastische und nicht elastische Bereich kann aus einem Gewebe, insbesondere Stoffgewebe, oder Gewirke oder aus Kunstleder oder natürlichem Leder bestehen. Der elastisch nachgiebige Bereich kann aus einem gummielastischen Gewebe oder stark dehnbaren Material oder aus einem Elasthan enthaltenden Gewebe bestehen.

Bei dieser Ausführungsform gemäß Figur 6 ist beispielsweise vorgesehen, dass der lösbare Verschluss 31 als Klettverschluss ausgebildet ist. Dieser lösbare Verschluss 31 umfasst eine Lasche 34 mit einem Hakenband 32 und eine Lasche 34 mit einem Schlaufenband 33 umfasst. Die jeweiligen Enden der Lasche 34, in welchen das Haken- und Schlaufenband 32, 33 miteinander verhaken, sind beispielsweise der Fixiertasche 18 zugeordnet. Die gegenüberliegenden Enden der jeweiligen Lasche 34 sind fest an dem unelastischen Bereich 51 des Fingerlings 14, 15, 16, 46, 47 befestigt. Dies kann durch Annähen und/oder Ankleben und/oder Verschweißen erfolgen. Es kann auch nur eine Lasche 34 vorgesehen sein, welche am jeweiligen Ende ein Haken- und Schlaufenband 32, 33 aufweist und im mittleren Bereich an dem unelastischen Bereich 51 des Fingerlings 14, 15, 16, 46, 47 oder an der Fixiertasche 18 fixiert ist. Bei diesen Ausführungsformen ist vorgesehen, dass beim Fixieren des Haken- und Schlaufenbandes 32, 33 der unelastische Bereich 21 und die Fixiertasche 18 aufeinander zubewegt werden, wodurch eine Streckung des Fingers in dem Fingerling 14, 15, 16, 46, 47 relativ zur Fixierschiene 24 erzielt wird.

In Figur 7 ist eine alternative Ausführungsform zu Figur 6 dargestellt. Der Aufbau des Fingerlings 14, 15, 16, 46, 47 mit der daran angeordneten Einstecktasche 18 entspricht der Ausführungsform gemäß Figur 6. Abweichend hierzu ist vorgesehen, dass das Haken- und Schlaufenband 32, 33 am jeweiligen Ende von einer einzigen Lasche 34 vorgesehen ist, wobei ein Ende der Lasche 34 beispielsweise an der Fixiertasche 18 oder an dem unelastischen Bereich 51 des Fingerlings 14, 15, 16, 46, 47 fest verbunden ist. Dadurch kann mittels einer Hand beispielsweise das Schlaufenband 33 zum Hakenband 32 fixiert werden. Eine Vertauschung ist ebenso unmöglich.

In Figur 8 ist eine alternative Ausführungsform des Therapiehandschuhs 11 zu Figur 1 dargestellt. Bei dieser Ausführungsform ist die Fixierschiene 24 an einer Handinnenseite lösbar fixiert. Die Fixiertaschen 18 sind auf der Innenseite der Finger vorgesehen. Das lösbare Befestigungsmittel 21 ist im Bereich der Handfläche vorgesehen.

Bei dieser Ausführungsform gemäß Figur 8 ist eine zweite Fixierschiene 41 vorgesehen. Diese Fixierschiene 41 weist einen Fingerschienenabschnitt 29 für einen Zeigefinger und einen Fingerschienenabschnitt 30 für einen Daumen auf. Der Handschuh 13 weist ergänzend einen Fingerling 46 für den Daumen und einen Fingerling 47 für den Zeigefinger auf. An jedem Fingerling 46, 47 ist bevorzugt eine Fixiertasche 18 vorgesehen. Die Fingerschienenabschnitte 29, 30 sind bevorzugt in Fixiertaschen 18 einsteckbar. Diesen gegenüberliegend ist eine Tasche oder ein lösbares Befestigungsmittel 21 vorgesehen, um diese Fixierschiene 41 im Bereich des Handballens zu fixieren.

An den Fingerlingen 46, 47 ist wie bei den Fingerlingen 14, 15, 16 jeweils ein lösbarer Verschluss 31 vorgesehen. Die Funktionsweise und die möglichen Ausführungsformen des lösbaren Verschlusses 31 entsprechen denjenigen, die zum Therapiehandschuh 11 gemäß den Figuren 1 bis 4 beschrieben sind.

Der Therapiehandschuh 11 kann aus einem Leder oder einem elastischen Kunststoffmaterial hergestellt sein, welches wie das Leder eine Perforation aufweisen kann. Alternativ kann das elastische Kunststoffmaterial auch atmungsaktiv ausgebildet sein.

## Patentansprüche

1. Therapiehandschuh zur Fixierung von zumindest einem Finger,
- mit einem Handschuh (13), der einen Zentralbereich (12) umfasst, welcher einen Handrücken und/oder eine Handfläche umschließt,
- mit zumindest einem von dem Zentralbereich (12) ausgehenden Fingerling (14, 15, 16, 46, 47), in dem der zu fixierende Finger aufgenommen werden kann,
- mit zumindest einer Fixierschiene (24, 41), welche einen Hauptschienenabschnitt (25) und daran angeordnet zumindest einen Fingerschienenabschnitt (26, 27, 28, 29, 30) aufweist,
- mit einem Befestigungsmittel (21) im Zentralbereich (12), durch welches die Fixierschiene (24, 41) lösbar befestigt ist, **dadurch gekennzeichnet, dass** der Therapiehandschuh mit zumindest einer an dem Fingerling (A) angeordneten Fixiertasche (18), in welche der Fingerschienenabschnitt (26, 27, 28, 29, 30) einsteckbar ist, und wobei die Fixierschiene (24, 41), welche in die Fingertasche (18) eingesetzt und durch das Befestigungsmittel (21) gehalten ist, den im Fingerling (14, 15, 16, 46, 47) aufgenommenen Finger in einer Therapiestellung relativ zum Handrücken fixiert, wobei
- an jedem eine Fixiertasche (18) aufweisenden Fingerling (14, 15, 16, 46, 47) ein lösbarer Verschluss (31) vorgesehen ist, durch welchen eine Anlageposition des Fingers im Fingerling (14, 15, 16, 46, 47) zum Fingerschienenabschnitt (26, 27, 28, 29, 30) einstellbar ist.

2. Therapiehandschuh nach Anspruch 1, **dadurch gekennzeichnet, dass** der Fingerling (14, 15, 16, 46, 47) zumindest abschnittsweise aus einem elastisch nachgiebigen Material ausgebildet ist und die Fixiertasche (18) aus einem, vorzugsweise gegenüber dem Material des Fingerlings (14, 15, 16, 46, 47), unelastischen Material besteht.

3. Therapiehandschuh nach Anspruch 2, **dadurch gekennzeichnet, dass** der Fingerling (14, 15, 16, 46, 47) einen der Fixiertasche (18) gegenüberliegenden Bereich (51) aus unelastischem Material aufweist, der zur Handinnenseite oder Handaußenseite ausgerichtet ist und zwischen dem unelastischen Bereich (51) des Fingerlings (14, 15, 16, 46, 47) und der Fixiertasche (18) ein elastisch nachgiebiger Bereich (53) ausgebildet ist.

4. Therapiehandschuh nach einem der vorhergehenden Ansprüche, **dadurch gekennzeichnet, dass** der lösbare Verschluss (31) im Bereich des ersten oder zweiten Fingergliedes oder im Bereich des Gelenkes zwischen dem ersten und zweiten Fingerglied oder das erste und zweite Fingerglied sowie das dazwischenliegende Gelenk übergreifend vorgesehen ist.

5. Therapiehandschuh nach einem der vorhergehenden Ansprüche, **dadurch gekennzeichnet, dass** der lösbare Verschluss (31) ein Klettverschluss ist.

6. Therapiehandschuh nach Anspruch 5, **dadurch gekennzeichnet, dass** der Klettverschluss ein Haken- und Schlaufenband (32, 33) aufweist und welche fest an dem unelastischen Bereich (51) des Fingerlings (14, 15, 16, 46, 47) oder der Fixiertasche (18) vorgesehen sind und gegenüberliegend an der Fixiertasche (18) oder dem unelastischen Bereich (51) zueinander fixierbar sind.

7. Therapiehandschuh nach Anspruch 5, **dadurch gekennzeichnet, dass** der Klettverschluss eine Lasche (34) aufweist, welche am jeweiligen Ende ein Haken- und Schlaufenband (32, 33) umfasst und dass ein Ende der Lasche (34) an der Fixiertasche (18) oder dem unelastischen Bereich (51) des Fingerlings (14, 15, 16, 46, 47) befestigt ist und die Lasche (34) den Fingerling (14, 15, 16, 46, 47) umschließend und das freie Ende der Lasche (34) an dem fixierten Ende der Lasche (34) befestigbar ist.

8. Therapiehandschuh nach einem der vorhergehenden Ansprüche, **dadurch gekennzeichnet, dass** die Fixierschiene (24, 41) an einem Handrücken anliegend oder an einer Handinnenseite anliegend in Fixiertaschen (18) befestigbar ist.

9. Therapiehandschuh nach Anspruch 1, **dadurch gekennzeichnet, dass** der zumindest eine Fingerschienenabschnitt (26, 27, 28, 29, 30) eine längliche Aussparung (36) aufweist, welche in der äußeren Kontur des Fingerschienenabschnitts (26, 27, 28, 29, 30) folgt und der Fingerschienenabschnitt (26, 27, 28, 29, 30) einen umlaufenden Steg (37) aufweist, oder welche in dem Fingerschienenabschnitt (26, 27, 28, 29, 30) in Längserstreckung eine oder zwei bauchige Konturen aufweist, welche im Bereich der Gelenke zwischen dem ersten und zweiten Fingerglied und/oder dem zweiten und dritten Fingerglied vorgesehen sind.

10. Therapiehandschuh nach einem der vorhergehenden Ansprüche, **dadurch gekennzeichnet, dass** im Hauptschienenabschnitt (25) der Fixierschiene (24) zumindest eine Freinehmung (39) vorgesehen ist.

11. Therapiehandschuh nach einem der vorhergehenden Ansprüche, **dadurch gekennzeichnet, dass** die Fixierschiene (24) als Stanzteil oder aus einem Kunststoff, insbesondere einem faserverstärkten Kunststoff, oder aus einem durch Wärme verformbaren Kunststoff hergestellt ist.

## Claims

1. Therapy glove for fixation of at least one finger,
- with a glove (13) comprising a central area (12) enclosing a back of the hand and/or a palm,
- with at least one finger stall (14, 15, 16, 46, 47) extending from the central region (12), in which the finger to be fixed can be accommodated,
- with at least one fixing rail (24, 41), which has a main rail section (25) and at least one finger rail section (26, 27, 28, 29, 30) arranged thereon,
- with a fastening means (21) in the central region (12), by means of which the fixing rail (24, 41) is detachably fastened,
**characterized in that**
- the therapy glove has at least one fixing pocket (18) arranged on the finger stall (A), into which the finger rail section (26, 27, 28, 29, 30) is insertable, and wherein the fixing rail (24, 41), which is inserted into the finger pocket (18) and held by the fastening means (21), fixes the finger received in the finger stall (14, 15, 16, 46, 47) in a therapy position relative to the back of the hand,
- wherein a releasable fastener (31) is provided on each finger stall (14, 15, 16, 46, 47) having a fixing pocket (18), by means of which a contact position of the finger in the finger stall (14, 15, 16, 46, 47) with respect to the finger rail section (26, 27, 28, 29, 30) is adjustable.

2. Therapeutic glove according to claim 1, **characterized in that** the finger stall (14, 15, 16, 46, 47) is formed at least in sections from an elastically yielding material and the fixing pocket (18) consists of a material which is preferably inelastic with respect to the material of the finger stall (14, 15, 16, 46, 47).

3. Therapeutic glove according to claim 2, **characterized in that** the finger stall (14, 15, 16, 46, 47) has a region (51) of inelastic material opposite the fixing pocket (18), which is oriented towards the inside or outside of the hand, and an elastically yielding region (53) is formed between the inelastic region (51) of the finger stall (14, 15, 16, 46, 47) and the fixing pocket (18).

4. Therapeutic glove according to one of the preceding claims, **characterized in that** the releasable closure (31) is provided in the region of the first or second phalanx or in the region of the joint between the first and second phalanx or overlapping the first and second phalanx and the joint located therebetween.

5. Therapeutic glove according to one of the preceding claims, **characterized in that** the releasable fastener (31) is a hook-and-loop fastener.

6. Therapeutic glove according to claim 5, **characterized in that** the hook-and-loop fastener has a hook-and-loop strap (32, 33) and which are firmly provided on the inelastic region (51) of the finger stall (14, 15, 16, 46, 47) or the fixing pocket (18) and fixable opposite one another on the fixing pocket (18) or the inelastic region (51).

7. Therapy glove according to claim 5, **characterized in that** the hook-and-loop fastener has a tab (34) which comprises a hook-and-loop tape (32, 33) at the respective end and one end of the tab (34) is attached to the fixing pocket (18) or the inelastic region (51) of the finger stall (14, 15, 16, 46, 47) and the tab (34) encloses the finger stall (14, 15, 16, 46, 47) and the free end of the tab (34) attachable to the fixed end of the tab (34).

8. Therapeutic glove according to one of the preceding claims, **characterized in that** the fixation splint (24, 41) is fastenable in fixation pockets (18) in contact with the back of a hand or in contact with the inside of a hand.

9. Therapeutic glove according to claim 1, **characterized in that** the at least one finger rail portion (26, 27, 28, 29, 30) has an elongate recess (36) which follows the outer contour of the finger rail portion (26, 27, 28, 29, 30) and the finger rail portion (26, 27, 28, 29, 30) has a circumferential web (37), or which has one or two bulbous contours in the finger splint portion (26, 27, 28, 29, 30) in longitudinal extension, which are provided in the region of the joints between the first and second phalanges and/or the second and third phalanges.

10. Therapeutic glove according to one of the preceding claims, **characterized in that** at least one free receiving means (39) is provided in the main rail section (25) of the fixation splint (24).

11. Therapeutic glove according to one of the preceding claims, **characterized in that** the fixing rail (24) is produced as a stamped part or from a plastic, in particular a fibre-reinforced plastic, or from a plastic which is deformable by heat.

## Revendications

1. Gant thérapeutique pour la fixation d'au moins un doigt,
- avec un gant (13) comprenant une zone centrale (12) qui entoure un dos de main et/ou une paume,
- avec au moins un doigtier (14, 15, 16, 46, 47) partant de la zone centrale (12), dans lequel le doigt à fixer peut être reçu,
- avec au moins un rail de fixation (24, 41), qui présente une section de rail principal (25) et, disposée sur celle-ci, au moins une section de rail à doigts (26, 27, 28, 29, 30),
- avec un moyen de fixation (21) dans la zone centrale (12), par lequel le rail de fixation (24, 41) est fixé de manière amovible,
**caractérisé en ce que**
- le gant thérapeutique comporte au moins une poche de fixation (18) disposée sur le doigtier (A), dans laquelle la section d'attelle de doigt (26, 27, 28, 29, 30) peut être insérée, et dans lequel le rail de fixation (24, 41), qui est insérée dans la poche de doigt (18) et est maintenue par le moyen de fixation (21), fixe le doigt reçu dans le doigtier (14, 15, 16, 46, 47) dans une position de thérapie par rapport au dos de la main,
- une fermeture amovible (31) étant prévue sur chaque doigtier (14, 15, 16, 46, 47) présentant une poche de fixation (18), grâce à laquelle une position d'appui du doigt dans le doigtier (14, 15, 16, 46, 47) par rapport au section de rail de doigt (26, 27, 28, 29, 30) peut être réglée.

2. Gant thérapeutique selon la revendication 1, **caractérisé en ce que** le doigtier (14, 15, 16, 46, 47) est réalisé, au moins par sections, dans un matériau élastiquement souple et **en ce que** la poche de fixation (18) est réalisée dans un matériau inélastique, de préférence par rapport au matériau du doigtier (14, 15, 16, 46, 47).

3. Gant thérapeutique selon la revendication 2, **caractérisé en ce que** le doigtier (14, 15, 16, 46, 47) présente une zone (51) en matériau non élastique opposée à la poche de fixation (18), qui est orientée vers la face intérieure ou extérieure de la main et une zone (53) élastiquement souple est formée entre la zone non élastique (51) du doigtier (14, 15, 16, 46, 47) et la poche de fixation (18).

4. Gant thérapeutique selon l'une des revendications précédentes, **caractérisé en ce que** la fermeture amovible (31) est prévue dans la zone de la première ou de la deuxième phalange ou dans la zone de l'articulation entre la première et la deuxième phalange ou chevauchant la première et la deuxième phalange ainsi que l'articulation située entre celles-ci.

5. Gant thérapeutique selon l'une des revendications précédentes, **caractérisé en ce que** la fermeture détachable (31) est une fermeture Velcro.

6. Gant thérapeutique selon la revendication 5, **caractérisé en ce que** la fermeture velcro présente une bande à crochets et à boucles (32, 33) et qui sont prévues de manière fixe sur la zone non élastique (51) du doigtier (14, 15, 16, 46, 47) ou de la poche de fixation (18) et peuvent être fixées l'une à l'autre en face de la poche de fixation (18) ou de la zone non élastique (51).

7. Gant thérapeutique selon la revendication 5, **caractérisé en ce que** la fermeture velcro présente une languette (34) qui comprend à chaque extrémité une bande à crochets et à boucles (32, 33) et **en ce qu'**une extrémité de la languette (34) est fixée à la poche de fixation (18) ou à la zone non élastique (51) du doigtier (14, 15, 16, 46, 47) est fixée et la languette (34) entoure le doigtier (14, 15, 16, 46, 47) et l'extrémité libre de la languette (34) peut être fixée à l'extrémité fixée de la languette (34) .

8. Gant thérapeutique selon l'une des revendications précédentes, **caractérisé en ce que** le rail de fixation (24, 41) peut être fixée dans des poches de fixation (18) en s'appuyant sur le dos d'une main ou en s'appuyant sur la face interne d'une main.

9. Gant thérapeutique selon la revendication 1, **caractérisé en ce que** l'au moins une section de rail de doigt (26, 27, 28, 29, 30) présente un évidement allongé (36) qui suit le contour extérieur de la section de rail de doigt (26, 27, 28, 29, 30) et la section de rail de doigt (26, 27, 28, 29, 30) présente une nervure périphérique (37), ou qui présente dans la section de rail de doigt (26, 27, 28, 29, 30), dans l'extension longitudinale, un ou deux contours bombés qui sont prévus dans la zone des articulations entre la première et la deuxième phalange et/ou la deuxième et la troisième phalange.

10. Gant thérapeutique selon l'une des revendications précédentes, **caractérisé en ce qu'**au moins un dégagement (39) est prévu dans la section de rail principale (25) de l'attelle de fixation (24).

11. Gant thérapeutique selon l'une des revendications précédentes, **caractérisé en ce que** le rail de fixation (24) est fabriquée sous forme de pièce découpée ou en une matière plastique, en particulier une matière plastique renforcée par des fibres, ou en une matière plastique déformable par la chaleur.
